# EUROPÄISCHE PATENTSCHRIFT

(11) **EP 1 837 036 B1**
(45) Veröffentlichungstag und Bekanntmachung des Hinweises auf die Patenterteilung: **24.11.2010**
(21) Anmeldenummer: 07090053.5
(22) Anmeldetag: 23.03.2007
(51) Int. Cl.: A61L 2/18, C09J 5/02, C09J 11/02, B43M 11/00, B43M 5/00

(54) **Schliessflüssigkeit**
Sealing liquid
Liquide de fermeture

(30) Priorität: 24.03.2006 DE 102006014164
(43) Veröffentlichungstag der Anmeldung: 26.09.2007
(73) Patentinhaber: Francotyp-Postalia GmbH, 16547 Birkenwerder (DE)
(72) Erfinder: Gerhardt, Thomas, 12249 Berlin (DE); Muhl, Wolfgang, 16540 Hohen Neuendorf (DE)
(74) Vertreter: Jungblut, Bernhard Jakob

(56) Entgegenhaltungen:
- EP-A- 1 616 716
- WO-A-2006/065544
- WO-A1-03/050222
- DE-A1- 4 243 477
- JP-A- 3 037 299
- US-A- 1 969 636
- US-A- 4 702 774
- US-A- 5 022 953
- US-A1- 2003 202 838
- US-B1- 6 696 399

## Beschreibung

### Gebiet der Erfindung.

Die Erfindung betrifft eine Schließflüssigkeit, insbesondere die Verandung einer Schlüßflüssigkeit zum Schließen von Poststücken, sowie Briefschließvorrichtungen und Frankiermaschinen enthaltend eine solche Schließflüssigkeit.

### Hintergrund der Erfindung und Stand der Technik.

Poststücke, insbesondere Briefe, werden bei hohem Aufkommen an zu versendenden Poststücken von einem Versender meist automatisch verschlossen und frankiert. Hierzu werden u.a. Schließvorrichtungen und Frankiermaschinen eingesetzt, wobei beide Komponenten oft mit einander verbunden sind und mitunter bauliche Einheiten bilden. Beispielhaft wird auf die Literaturstelle DE 20 2004 011 390 U1 verwiesen.

Im Zusammenhang mit dem Schließen von Poststücken, deren Verschlusslaschen mit einer Gummierung versehen sind, ist es erforderlich, die Gummierung vor dem Schließen anzufeuchten. Hierzu wird eine Schließflüssigkeit (automatisch) auf die Gummierung aufgetragen. In diesen Zusammenhängen stellen sich eine Mehrzahl von technologischen Problemen. Zum ersten muss die Schließflüssigkeit die Gummierung hinreichend benetzen. Bei nicht hinreichender Benetzung perlt die Schließflüssigkeit ab und letztendlich wird keine brauchbare Klebung erhalten. Zum zweiten muss die auf die Gummierung aufgebrachte Menge an Schließflüssigkeit so bemessen sein, dass einerseits die Gummierung klebt, jedoch andererseits ein unerwünschtes Verwaschen oder Verwischen überschüssiger Schließflüssigkeit auf der Poststückhülle nach dem Verschließen unterbleibt. Letzteres ist wichtig insbesondere für die Vermeidung des Verwaschens oder Verwischens von wasserempfindlichen Aufschriften oder Aufdrucken, wie Frankierstempelabdrucken, auf dem Poststück oder darunter oder darüber liegenden Poststücken. Hieraus folgt, dass die Dosierung der Schließflüssigkeit eine kritische Größe im Schließprozess ist.

Hinzu kommt, dass Schließvorrichtungen systembedingt mit zu viel Wasser starten, wenn ein Schwamm der Schließvorrichtung bei Einschalten der Schließvorrichtung mit der Schließflüssigkeit gefüllt wird. Erst nach einer Anlaufanzahl von geschlossenen Poststücken stellt sich im weiteren (quasi-) kontinuierlichen Betrieb ein Gleichgewicht hinsichtlich der im Schwamm enthaltenen Menge an Schließflüssigkeit ein. Im Ergebnis wird in der Regel am Anfang einer Serie von Schließungen die Dosis an Schließflüssigkeit zu hoch sein, da bei Abstimmung auf eine anfänglich zutreffende Dosierung die Gleichgewichtsdosierung wiederum zu niedrig wäre. Gerade bei den ersten Poststücken besteht daher die erhebliche Gefahr des Überschusses an Schließflüssigkeit mit der Folge der Gefahr des Verwaschens von wasserempfindlichen Aufdrucken, wie Frankierstempelaufdrucken.

Weiterhin problematisch ist, dass in der Praxis einer Schließvorrichtung Poststücke, insbesondere Briefe, zugeführt werden, deren Poststückhülle bzw. Briefumschlag aus Papierwerkstoffen mit verschiedenen Materialeigenschaften besteht. So können Papierwerkstoffe einer niedrigen Dichte erheblich saugfähiger sein, als Papierwerkstoffe mit hoher Dichte. Für eine zuverlässige und reproduzierbare Schließung auch von Poststücken mit verschiedenen Papierwerkstoffen der Poststückhülle wäre es daher notwendig, die Menge an Schließflüssigkeit nach Maßgabe des betreffenden Papierwerkstoffes zu dosieren. Bei einem Werkstoff mit hoher Saugfähigkeit wäre eine vergleichsweise hohe Menge an Schließflüssigkeit notwendig, da ansonsten die Schließflüssigkeit schnell aufgesaugt wäre und die Klebung nicht hinreichend ausfallen würde. Bei einem Werkstoff mit niedriger Saugfähigkeit wäre eine vergleichsweise geringe Menge an Schließflüssigkeit notwendig, da ansonsten die Schließflüssigkeit nicht hinreichend aufgesaugt würde und ein Verwaschen und Verlaufen überschüssiger Schließflüssigkeit drohen würde mit den vorstehend beschriebenen nachteiligen Effekten.

Aus der Praxis sind verschiedene Schließflüssigkeiten bekannt. Dies sind wäßrige Lösungen, die einerseits Biozide und Fungizide enthalten können, um die Entstehung von Keimkolonien und unangenehmen Gerüchen zu verhindern. Andererseits kann ein Benetzungsmittel beigegeben sein. In der Praxis wird auch von Anwendern beispielsweise Spülmittel als Benetzungsmittel zugegeben.

Die insofern bekannten Schließflüssigkeiten genügen allen den Anforderungen, da eine für alle Poststücke geeignete Dosierung, und zwar von Anfang an, praktisch nicht einstellbar ist und je nach Anzahl und Art der Poststücke eine Mehrzahl von unzureichenden Schließungen und/oder Verwaschungen zu befürchten sind.

Des Weiteren werden in einem anderen technischen Bereich, der Herstellung und Schließung von Kartons gummierte Paketbänder bzw. Montagebänder verwendet, mittels welchen Kartons verschlossen bzw. in Behälterform fixiert werden. Die Anbringung dieser Paketbänder bzw. Montagebänder erfolgt oft auch automatisiert, so dass sich grundsätzlich die vorstehend beschriebenen Probleme in diesem Bereich stellen.

Bekannt sind auch Mittel zum Befeuchten einer Gummierung: US-A-1969636 offenbart ein Gemisch aus Wasser, Alkohol und Glykol für ein solches Befeuchten.

Technisches Problem der Erfindung.

Der Erfindung liegt daher das technische Problem zu Grunde, eine Schließflüssigkeit anzugeben, mit welcher bei einer definierten Dosierungseinstellung alle zugeführten Poststücke, und zwar von Anfang an, mit verbesserter Zuverlässigkeit geschlossen werden können.

Grundzüge der Erfindung und bevorzugte Ausführungsformen.

Zur Lösung dieses technischen Problems lehrt die Erfindung die Verwendung einer Schließflüssigkeit zum Schließen von Poststücken wie in Anspruch 1 definiert.

Ein Penetrationsmittel ist eine Substanz, die zu einer gegenüber Wasser verkürzten, insbesondere um zumindest 10%, vorzugsweise zumindest 20%, höchstvorzugsweise zumindest 50%, bezogen auf die Wegschlagzeit von reinem Wasser, verkürzten Wegschlagzeit der Schließflüssigkeit in Papierwerkstoffen führt. Benetzungsmittel sind keine Penetrationsmittel im Sinne der Erfindung. Die Wegschlagzeit wird als die Zeit gemessen, in welcher ein auf den Papierwerkstoff aufgebrachter Tropfen der Schließflüssigkeit eines Volumens von 100 pl ± 5 pl in das Papier vollständig eingedrungen ist. Die Messung der Wegschlagzeit, auch als Penetrationszeit bekannt, ist aus der Tintenstrahldrucktechnologie bekannt und hierzu wird lediglich beispielsweise auf die Literaturstelle EP 1072653 A2 verwiesen.

Es wurde gefunden, dass grundsätzlich alle Penetrationsmittel im Rahmen der Erfindung einsetzbar sind, die auch in der Tintenstrahldrucktechnologie für wäßrige Tinten eingesetzt werden.

Mit der Erfindung wird erreicht, dass eine Schließvorrichtung einmal auf eine Grundeinstellung der Dosierung eingestellt werden muss, wobei diese Grundeinstellung die in der Praxis unter allen in Frage kommenden Betriebszuständen größte für eine zuverlässige Schließung benötigte Dosis sein kann (Umschlag mit höchster Saugkraft bei eingestelltem Gleichgewicht der Befüllung des Schwamms). Zwar wird dann in einer Mehrzahl von Schließungen eine Überdosierung, bezogen auf die für eine gute Klebung benötigte Menge, an Schließflüssigkeit erfolgen, dies führt jedoch nicht zu den eingangs beschriebenen Verwaschungen, da die überschüssige Schließflüssigkeit unter Wirkung des Penetrationsmittels schnell in den Papier- oder Pappwerkstoff der Poststückhülle penetriert. Im Ergebnis werden alle der Schließvorrichtung zugeführten Poststücke mit dieser Grundeinstellung zuverlässig geschlossen, ohne dass unerwünschte Verwaschungen entstehen, und zwar unabhängig vom Werkstoff der Poststückhülle oder ob eine Schließungsserie gerade anläuft oder schon fortgeschritten ist.

Geeignete Penetrationsmittel sind beispielsweise C1-8 Alkylester von C1-8 Mono-, Di-, oder Tricarbonsäuren, wobei die Carbonsäure OH-substituiert, insbesondere in α-Stellung, sein kann. Die Carboxylgruppen können vollständig oder teilweise verestert sein. Andere bevorzugte Penetrationsmittel sind C1-8 Ether von C1-8 Polyolen, wobei zumindest eine OH-Gruppe des Polyols nicht verethert ist. Als Polyole kommen insbesondere Diole oder Triole in Frage, wobei in letzterem Falle 1 oder 2 OH-Gruppen verethert sein können. C1-8 Alkyl kann linear, verzweigt, gesättigt oder ungesättigt sein. Als C1-8 Alkyl kommen beispielsweise in Frage. Methyl, Ethyl, Propyl, Isopropyl, Butyl, Isobutyl, tert. Butyl, Pentyl usw.. C1-8 Carbonsäuren können linear oder verzweigt, gesättigt oder ungesättigt sein. Als C1-8 Carbonsäuren kommen in Frage Ameisensäure, Essigsäure, Propionsäure, Buttersäure, Valeriansäure, Capronsäure, Isobuttersäure, Isovaleriansäure, Acrylsäure, Crotonsäure usw.. Als Hydroxycarbonsäuren kommen beispielsweise in Frage Glykolsäure, Milchsäure, Mandelsäure, Äpfelsäure, Weinsäure und Citronensäure. Als Diole kommen beispielsweise in Frage 1,2-Ethandiol, 1,3-Propandiol, 1,2-Propandiol, 1,4-Butandiol, 1,3-Butandiol, 1,2-Butandiol, usw.. Das Polyol kann eine oder mehrere Ethergruppen enthalten. Ein Beispiel für ein solches Polyol ist 2,2'-Oxydiethanol (Diethylenglykol). Als geeignete konkrete Verbindungen werden nicht abschließend genannt: Methyllactat, Ethyllactat, Propyllactat, Isopropyllactat, Äpfelsäurediester, Äpfelsäuremonoester, Weinsäurediester, Weinsäuremonoester, Ethylenglycolmonomethylether, Ethylenglycolmonoethylether, Ethylenglycol-monopropylether, Ethylenglycolmonobutylether, Diethylenglycolmonomethylether, Diethylenglycolmonoethylether, Diethylenglycolmonopropylether, Diethylenglycolmonobutylether.

Bezüglich weiterer geeigneter Penetrationsmittel wird lediglich beispielhaft auf die Literaturstellen EP 1072653 A und die darin genannten Literaturstellen, sowie die Literaturstellen DE 69628897 T2, US-5,674,314 A verwiesen.

Die erfindungsgemäß verwendete Schließflüssigkeit enthält ein tensidisches Benetzungsmittel. Das Benetzungsmittel kann ausgewählt sein aus der Gruppe bestehend aus "nichtionische Tenside, amphotere Tenside, anionische Tenside, kathionische Tenside, Ricinoleamidpropyl Betaine". Vorzugsweise handelt es sich um biologisch abbaubare Tenside. Bevorzugt werden amphotere Tenside eingesetzt, da damit überraschenderweise eine besonders gute Benetzung der Gummierung gelingt.

Des weiteren kann eine Schließflüssigkeit einen oder mehrere der folgenden Zusatz- und/oder Hilfsstoffe enthalten: Stabilisierer und/oder Puffer, Farbstoffe, und/oder Aromastoffe. Selbstverständlich können auch Biozide (beispielsweise Parmetol A6 oder Cetyltrimethylammoniumbromid) und/oder Fungizide in üblicher Weise zugesetzt sein. Als Farbstoffe und Aromastoffe kommen vorzugsweise Substanzen mit Zulassung als Lebensmittelzusatzstoffe in Frage, wie Lebensmittelfarben, Citral, Hexylhexonat usw.. Stabilisierer und/oder Puffer stabilisieren Bestandteile der Schließflüssigkeit, die hydrolysiert werden können. Beispielsweise ist NaLactat ein geeigneter Stabilisierer bzw. Puffer für Lactatester. Als Stabilisierer bzw. Puffer kommen insbesondere alle üblichen Stellsalze, wie Na-Lactat, NaCl, KCl usw., in Frage.

Eine erfindungsgemäß verwendete Schließflüssigkeit hat vorzugsweise folgende Zusammensetzung: a) 50 bis 99,9 Gew.-% Wasser, b) 0,1 bis 50,0 Gew.-% Penetrationsmittel, c) 0 bis 10,0 Gew.-% Benetzungsmittel, d) 0 bis 10,0 Gew.-% Stabilisierer und/oder Puffer, e) 0 bis 5,0 Gew.-% Farbstoffe, f) 0 bis 1,0 Gew.-% Aromastoffe, und g) 0 bis 10,0 Gew.-% üblicher Hilfsstoffe, wobei sich die Komponenten a) bis g) stets zu 100 Gew.-% addieren. Insbesondere können eingerichtet sein: a) 70 bis 99,0, insbesondere 80 bis 99,0, Gew.-% Wasser, b) 1,0 bis 30,0, insbesondere 1,0 bis 20, Gew.-% Penetrationsmittel, c) 0 bis 3,0, insbesondere 0,01 bis 1,0, Gew.-% Benetzungsmittel, d) 0 bis 3,0, insbesondere 0,01 bis 2,0, Gew.-% Stabilisierer und/oder Puffer, e) 0 bis 2,0, insbesondere 0,00001 bis 1,0, Gew.-% Farbstoffe, f) 0 bis 1,0, insbesondere 0,00001 bis 0,001, Gew.-% Aromastoffe und g) 0 bis 1,0, insbesondere 0 bis 0,1 Gew.-% Biozide und/oder Fungizide und/oder sonstige üblichen Hilfsstoffe.

Eine besonders bevorzugt verwendete Schließflüssigkeit besteht aus 0,5 bis 10 Gew.-%, insbesondere 1 bis 5 Gew.-%, Penetrationsmittel, insbesondere einen C1-4 Alkylester einer C1-C4 α-Hydroxycarbonsäure, beispielsweise Ethyllactat, 0,1 bis 2 Gew.-%, insbesondere 0,2 bis 1 Gew.-% eines tensidischen Benetzungsmittels, insbesondere eines amphoteren Tensides, 0 bis 5 Gew.-%, insbesondere 0,5 bis 2 Gew.-%, eines Stabilisierers, beispielsweise NaLactat, und 0 bis 2 Gew.-%, insbesondere 0,00001 bis 0,1 Gew.-% weiterer Zusatz- oder Hilfsstoffe, wie Farbstoffe, Biozide, Fungizide und/oder Aromastoffe.

Die Erfindung betrifft die Verwendung einer vorstehend beschriebenen Schließflüssigkeit zum Verschließen von Poststücken, wobei eine mit einer Gummierung versehene Lasche einer das Poststück umhüllenden Poststückhülle mit der Schließflüssigkeit befeuchtet wird, und wobei die benetzte Lasche dann umgeklappt und an einer gegenüberliegenden Fläche der Poststückhülle angedrückt und so das Poststück verschlossen wird. Das Poststück kann insbesondere ein Brief und die Poststückhülle ein Briefumschlag sein. Bevorzugt ist es, wenn die Befeuchtung der Gummierung sowie das Umklappen und Andrücken der Lasche in einer Briefschließvorrichtung automatisch gesteuert erfolgt. Rein vorsorglich sei aber auch darauf hingewiesen, dass die erfindungsgemäßen Vorteile sich grundsätzlich auch bei einer manuellen Applikation der Schließflüssigkeit ergeben.

Die Erfindung betrifft des weiteren die Verwendung einer vorstehend beschriebenen Schließflüssigkeit zum Fixieren und/oder Schließen einer Kartonverpackung, wobei ein mit einer Gummierung versehenes Klebeband mit der Schließflüssigkeit befeuchtet wird, und wobei das befeuchtete Klebeband auf der Kartonverpackung angebracht wird. Es versteht sich, dass die Seite des Klebebandes mit der Gummierung auf den Karton angedrückt wird.

Die Erfindung betrifft des weiteren die Verwendung der Schließflüssigkeit in einer Briefschließvorrichtung mit einem Tank, enthaltend eine erfindungsgemäß verwendete Schließflüssigkeit, und mit einer Befeuchterschwinge, welche zur Abnahme von Schließflüssigkeit aus dem Tank und Übertragung abgenommener Schließflüssigkeit auf die Gummierung eines in die Briefschließvorrichtung eingelegten Poststückes eingerichtet ist. Eine geeignete Briefschließvorrichtung ist beispielsweise in der Literaturstelle DE 20 2004 011 390 U1 beschrieben. Die Erfindung betrifft schließlich die Verwendung der Schließflüssigkeit in einer Frankiervorrichtung mit einer vorstehend beschriebenen Briefschließvorrichtung.

Im Folgenden wird die Erfindung anhand von lediglich Ausführungsformen darstellen Beispielen näher erläutert.

### Beispiel 1: Messung der Schließzeit

Verschiedene Schließflüssigkeiten wurden in den folgend beschriebenen Experimenten untersucht. Verwendet wurde eine Frankiermaschine Jetmail der Firma Francotyp-Postalia GmbH (DEU V 8.64A) mit gebrauchtem Powersealer. Zum einfachen Wechsel der Schließflüssigkeit wurde die Schlauchzufuhr vom Vorratsbehälter des Powersealers entfernt und in ein 10 ml Becherglas mit der zu prüfenden Schließflüssigkeit gehalten. Bei einem Wechsel der Schließflüssigkeit wurde zusätzlich zum Spülen der Schläuche (Service Mode) und der Schwammaufnahme immer ein neuer Schwamm verwendet.

Die vom Briefumschlag benötigte Zeit vom Schließer bis zum Powersealer beträgt etwa 1,5 s. Der durch das Handling bedingte Zeitpunkt der Verklebungsprüfung beträgt ab Powersealer etwa 0,5 s. Die gesamte Zeit ab Befeuchtung beträgt somit mindestens 2 s. Am Powersealer wird die befeuchtete Lasche nur für einen Bruchteil einer Sekunde an den Umschlag angedrückt. Bestimmt wurde die ab der Benetzung der Lasche vergangene Zeit, ab welcher die Umschlagslasche nicht mehr zerstörungsfrei ablösbar ist.

### Beispiel 2: Messung der Wegschlagzeit.

Die Wegschlagzeit bzw. Penetrationszeit der getesteten Schließflüssigkeiten erfolgte, indem ein 0,5 µl Tropfen, dosiert aus einer Einmal-Kapillarpipette (DESAGA GmbH, Deutschland, Artikelnr. 120192), auf einen Briefumschlag gesetzt wurde. Der tropfen hat typischerweise je nach Oberflächenspannung und/oder Benetzung einen Durchmesser von 1 bis 2 mm. Gemessen wird die Zeit beginnend mit dem Aufsetzen des Tropfens bis zum vollständigen Eindringen der Schließflüssigkeit in das Papier, was durch verschwindenden Glanz erkennbar ist. Zum Zwecke der besseren Erkennung war dem getesteten Schließflüssigkeiten 0,04 Gew.-% Farbstoff (Säurerhodamin, Duasyn) beigemischt.

### Beispiel 3: Durchführung und Ergebnisse der Messungen für erfindungsgemäße Schließflüssigkeiten und Vergleichs-Schließflüssigkeiten.

Getestet wurde die folgenden Briefumschläge, welche aus deutlich unterschiedlichen Papierwerkstoffen bestehen. Als Briefumschlag 1 ist bezeichnet OEKO-PIROL 90 Weiß, Fenster (229 x 324)mm, Best. Nr. 71126/06/C4 2279 "OF Kuvermatic", Otto Ficker AG, Deutschland. Als Briefumschlag 22 ist bezeichnet "Bless Of recycling", Fenster (235 x 125)mm, Best. Nr. 7140383-3602203, Umweltzeichen 14/9823, 100% Altpapier. Als Briefumschlag 108 ist bezeichnet "POSTHORN" weiß, Fenster C6/5 (229 x 114)mm, Best. Nr. 2526149, Bong (ehemals Schmidt Papier), Deutschland. Als Briefumschlag 208 ist bezeichnet "awamatic" highspeed weiß, ohne Fenster (229 x 324)mm, Art. Nr. 049080, Automatenhüllen der August Wegener GmbH & Co, Deutschland.

Als käuflich erwerbbare Schließflüssigkeiten wurden zu Vergleichszwecken die folgenden Produkte eingesetzt: Schließflüssigkeit "FT" der Firma FrancoTech GmbH, Deutschland, "E-Z Seal" der Firma Pitney Bowes, USA, und "Quick Seal"-Zubereitung der Firma Service Industries, USA, in der in Kanada vertriebenen Form.

Als Penetrationsmittel wurden getestet: EGDA (Ethylenglycoldiactet), DEGMEE (Diethylenglycolmonoethylether), EL (Ethyllactat) und EGMBE (Ethylenglycolmonobutylether. Alle diese Substanzen sind von der Firma Merck, Deutschland, erhältlich.

SW bezeichnet eine erfindungsgemäß verwendete Schließflüssigkeit.

Als Hilfsmittel wurden eingesetzt: NaLac (Natriumlactat Lösung 50%, Merck, Deutschland), TMN 6 (Tergitol TMN 6 nichtionisches Tensid, Fluka, Deutschland), AM R 40 (Ricinoleamidepropyl Betaine, amphoteres Tensidmuster, Degussa, Deutschland) und Säurerhodamin BC 01 als Farbstoff (Duasyn Merck).

In der Tabelle I sind einige Messwerte zur Oberflächenspannung (gemessen mit KRÜSS K10ST Ring-Tensiometer) sowie der Wegschlagzeit dargestellt, die auf Briefumschlag 108 erhalten wurden. Man erkennt, dass keine Korrelation zwischen Oberflächenspannung und Wegschlagzeit besteht. Dies belegt, dass Benetzungsmittel nicht per se auch als Penetrationsmittel geeignet sind und Penetrationsmittel als Komponente wichtig sind. Des weiteren erkennt man, dass die handelsüblichen Produkte FT und Quick Seal Wegschlagzeiten, wie Wasser aufweisen, während erfindungsgemäß verwendete Schließflüssigkeiten (DGMEE bis SW) demgegenüber stark reduzierte Wegschlagzeiten aufweisen.

In der Tabelle II sind Wegschlagzeiten für verschiedene Briefumschläge dargestellt. Man erkennt, dass mit erfindungsgemäß verwendeten Schließflüssigkeiten gegenüber den handelsüblichen Schließflüssigkeiten durchweg stark verkürzte Wegschlagzeiten erhalten werden, und zwar auf allen getesteten Briefumschlägen (n.e. = nicht ermittelt, 301 = Abbruch der Messung nach 5 min.).

Schließlich wurde die Benetzung der Gummierung geprüft, wobei für alle Schließflüssigkeiten mit TMN 6 oder AM R 40 durchweg gute bis sehr gute Benetzung festgestellt wurde.

Insgesamt sehr kurze Wegschlagzeiten werden bei Kombination des Penetrationsmittels mit einem Benetzungsmittel erreicht.

In der Tabelle III sind Schließzeiten einer erfindungsgemäß verwendeten Schließflüssigkeit im Vergleich mit Wasser dargestellt. Die erfindungsgemäß verwendete Schließflüssigkeit ist 2% EL, 0,5% AM R 40 und 1,2 % NaLac. Unendlich bezeichnet eine innerhalb der Messzeit nicht erfolgte Schließung. Gemessen wurde bei 25°C und 21% relative Luftfeuchtigkeit.

**Tabelle I**

| Probe | Oberflächensp. [mN/m] | Wegschlagzeit [s] |
|---|---|---|
| VE-Wasser | 71,5 | 301 |
| FT | 56, 8 | 301 |
| Quick Seal | 47,5 | 301 |
| DEGMEE 20% | 48,3 | 33 |
| EL 6% | 49,1 | 19 |
| EGDA 15% | 39,5 | 2,1 |
| SW | 35,1 | 14 |
| AM R 40 0,5% | 35,1 | 45 |

**Tabelle II**

| Probe | Wegschlagzeiten [s] für versch. Umschläge | | | |
|---|---|---|---|---|
| | 1 | 22 | 108 | 208 |
| VE-Wasser | 301 | 301 | 301 | 301 |
| FT | 301 | 301 | 301 | n.e. |
| Quick Seal | 301 | 301 | 301 | n.e. |
| E-Z Seal | 301 | 301 | 301 | n.e. |
| EGDA 5% | 97 | 184 | 35 | n.e. |
| EGDA 7% | 12 | 41 | 4,8 | n.e. |
| EGDA 10% | 2 | 17 | 4 | n.e. |
| EGDA 13% | 1,5 | 3,7 | 2 | n.e. |
| EGDA 15% | 1 | 4,7 | 2,1 | n.e. |
| DEGMEE 5% | 301 | 301 | 301 | n.e. |
| DEGMEE 10% | 165 | 139 | 89 | n.e. |
| DEGMEE 15% | 113 | 135 | 82 | n.e. |
| DEGMEE 20% | 30 | 46 | 33 | n.e. |
| EL 3%, NaLac 1,2% | 211 | 255 | 55 | n.e. |
| EL 6%, NaLac 1,2% | 108 | 134 | 19 | n.e. |
| EL 9%, NaLac 1,2% | 20 | 25 | 8 | n.e. |
| EL 12%, NaLac 1,2% | 5 | 19 | 5 | n.e. |
| EL 3,3%/NaLac 1,2%/TMN 6 0,5% | 0,7 | 1,1 | 0,9 | n.e |
| EL 2,0%/NaLac 1,2%/AM R 40 0,5% | 35 | 24 | 14 | 34 |
| EL 2,0%/AM R 40 0,5% | 35 | 31 | 24 | 47 |

**Tabelle III**

| Umschlag | Wasser | Schließflüssigkeit |
|---|---|---|
| 1 | unendlich | 10 s |
| 22 | 10 s | 5 s |
| 108 | 10 s | 10 s |
| 208 | 8 s | 5 s |

## Patentansprüche

1. Verwendung einer Schließflüssigkeit enthaltend Wasser, ein Benetzungsmittel und ein Penetrationsmittel, wobei das Penetrationsmittel ein C1-8 Alkylester von C1-8 Mono-, Di-, oder Tricarbonsäuren, wobei die Carbonsäure optional OH-substituiert, insbesondere in α-Stellung OH-substituiert, sein kann, oder ein C1-8 Ether von C1-8 Polyolen, wobei zumindest eine OH-Gruppe des Polyols nicht verethert ist, ist und wobei das Benetzungsmittel ein tensidisches Benetzungsmittel ist zum Verschließen von Poststücken, wobei eine mit einer Gummierung versehene Lasche einer das Poststück umhüllenden Poststückhülle mit der Schließflüssigkeit befeuchtet wird, und wobei die benetzte Lasche dann umgeklappt und an einer gegenüberliegenden Fläche der Poststückhülle angedrückt wird oder zum Fixieren und/oder Schließen einer Kartonverpackung, wobei ein mit einer Gummierung versehenes Klebeband mit der Schließflüssigkeit befeuchtet wird, und wobei das befeuchtete Klebeband auf der Kartonverpackung angebracht wird.

2. Verwendung nach Anspruch 1, wobei das Penetrationsmittel ausgewählt ist aus der Gruppe bestehend aus Methyllactat, Ethyllactat, Propyllactat, Isopropyllactat, Äpfelsäurediester, Äpfelsäuremonoester, Weinsäurediester, Weinsäuremonoester, Ethylenglycolmonomethylether, Ethylenglycolmonoethylether, Ethylenglycol-monopropylether, Ethylenglycolmonobutylether, Diethylenglycolmonoethylether, Diethylenglycolmonoethylether, Diethylenglycolmonopropylether, und Diethylenglycolmonobutylether.

3. Verwendung nach Anspruch 1 oder 2, wobei das Benetzungsmittel ausgewählt ist aus der Gruppe bestehend aus nichtionische Tenside, anionische Tenside, kathionische Tenside, amphotere Tenside, Ricinoleamidpropyl Betaine.

4. Verwendung nach einem der Ansprüche 1 bis 3, wobei die Schließflüssigkeit zusätzlich enthalten einen oder mehrere der folgenden Zusatz- und/oder Hilfsstoffe:
a) Stabilisierer und/oder Puffer,
b) Farbstoffe, und
c) Aromastoffe.

5. Verwendung nach einem der Ansprüche 1 bis 4 mit
a) 50 bis 99,9 Gew.-% Wasser,
b) 0,1 bis 50,0 Gew.-% Penetrationsmittel,
c) 0,01 bis 10,0 Gew.-% Benetzungsmittel,
d) 0 bis 10,0 Gew.-% Stabilisierer und/oder Puffer,
e) 0 bis 5,0 Gew.-% Farbstoffe,
f) 0 bis 5,0 Gew.-% Aromastoffe, und
g) 0 bis 10,0 Gew.-% üblicher Hilfsstoffe,
wobei sich die Komponenten a) bis f) stets zu 100 Gew.% addieren.

6. Verwendung nach einem der Ansprüche 1 bis 5 mit
a) 70 bis 99,0, insbesondere 80 bis 99,0, Gew.-% Wasser,
b) 1,0 bis 30,0, insbesondere 1,0 bis 20, Gew.-% Penetrationsmittel,
c) 0,01 bis 3,0, insbesondere 0,01 bis 1,0, Gew.-% Benetzungsmittel,
d) 0 bis 3,0, insbesondere 0,01 bis 2,0, Gew.-% Stabilisierer und/oder Puffer,
e) 0 bis 2,0, insbesondere 0,01 bis 1,0, Gew.-% Farbstoffe,
f) 0 bis 1,0, insbesondere 0,01 bis 0,1, Gew.-% Aromastoffe, und
g) 0 bis 1,0, insbesondere 0 bis 0,1 Gew.-% Biozide und/oder Fungizide und/oder sonstige üblichen Hilfsstoffe,
wobei sich die Komponenten a) bis f) stets zu 100 Gew.% addieren.

7. Verwendung nach einem der Ansprüche 1 bis 6, wobei das Poststück ein Brief und die Poststückhülle ein Briefumschlag ist.

8. Verwendung nach einem der Ansprüche 1 bis 7, wobei die Befeuchtung der Gummierung sowie das Umklappen und Andrückern der Lasche in einer Briefschließvorrichtung automatisch gesteuert erfolgt.

9. Verwendung nach einem der Ansprüche 1 bis 6, wobei die Schließflüssigkeit in einer Briefschließvorrichtung mit einem Tank und mit einer Befeuchterschwinge verwendet wird, wobei die Befeuchterschwinge zur Abnahme von Schließflüssigkeit aus dem Tank und Übertragung abgenommener Schließflüssigkeit auf die Gummierung eines in die Briefschließvorrichtung eingelegten Poststückes eingerichtet ist.

10. Verwendung nach einem der Ansprüche 1 bis 6, wobei die Schließflüssigkeit in einer Frankiervorrichtung mit einer Briefschließvorrichtung mit einem Tank und mit einer Befeuchterschwinge verwendet wird, wobei die Befeuchterschwinge zur Abnahme von Schließflüssigkeit aus dem Tank und Übertragung abgenommener Schließflüssigkeit auf die Gummierung eines in die Briefschließvorrichtung eingelegten Poststückes eingerichtet ist.

## Claims

1. The use of a sealing liquid comprising water, a moistening agent and a penetration agent, wherein the penetration agent may be a C1-8 alkyl ester of C1-8 mono-, di- or tricarboxylic acids, wherein the carboxylic acid optionally may be OH-substituted, in particular OH-substituted in the α position, or is a C1-8 Ether of C1-8 polyols, wherein at least one OH-group of the polyol is not etherized, and wherein the moistening agent is a surface-active moistening agent for sealing postal items, wherein a flap provided with a gumming of a postal item envelope enclosing the postal item is wetted with the sealing liquid, and wherein the wetted flap is then folded back and pressed on an opposite surface of the postal item envelope or is used for fixing and/or sealing a carton package, wherein a sealing tape provided with a gumming is wetted with the sealing liquid, and wherein the wetted sealing tape is applied on the carton package.

2. The use according to claim 1, wherein the penetration agent is selected from the group consisting of methyl lactate, ethyl lactate, propyl lactate, isopropyl lactate, malic acid diester, malic acid monoester, tartaric acid diester, tartaric acid monoester, ethylene glycol monomethyl ether, ethylene glycol monoethyl ether, ethylene glycol monopropyl ether, ethylene glycol monobutyl ether, diethylene glycol monoethyl ether, diethylene glycol monoethyl ether, diethylene glycol monopropyl ether, diethylene glycol monobutyl ether.

3. The use according to claim 1 or 2, wherein the moistening agent is selected from the group consisting of non-ionic surfactants, anionic surfactants, cationic surfactants, amphoteric surfactants, ricin oleamide propyl betaines.

4. The use according to one of claims 1 to 3, wherein the sealing liquid additionally contains one or several of the following additional and/or auxiliary agents:
a) stabilizers and/or buffers,
b) colorants, and
c) flavoring agents.

5. The use according to one of claims 1 to 4 with
a) 50 to 99.9 wt.% water,
b) 0.1 to 50.0 wt.% penetration agent,
c) 0.01 to 10.0 wt.% moistening agent,
d) 0 to 10.0 wt.% stabilizers and/or buffers,
e) 0 to 5.0 wt.% colorants,
f) 0 to 5.0 wt.% flavoring agents, and
g) 0 to 10.0 wt.% usual auxiliary agents,
wherein the sum of the components a) to f) always totals 100 wt.%.

6. The use according to one of claims 1 to 5 with
a) 70 to 99.0, in particular 80 to 99.0 wt.% water,
b) 1.0 to 30.0, in particular 1.0 to 20 wt.% penetration agent,
c) 0.01 to 3.0, in particular 0.01 to 1.0 wt.% moistening agent,
d) 0 to 3.0, in particular 0.01 to 2.0 wt.% stabilizers and/or buffers,
e) 0 to 2.0, in particular 0.01 to 1.0 wt.% colorants,
f) 0 to 1.0, in particular 0.01 to 0.1 wt.% flavoring agents and
g) 0 to 1.0, in particular 0 to 0.1 wt. % biocides and/or fungicides and/or other usual auxiliary agents,
wherein the sum of the components a) to f) always totals 100 wt.%.

7. The use according to one of claims 1 to 6, wherein the postal item is a letter and the postal item envelope is a letter envelope.

8. The use according to one of claims 1 to 7, wherein the moistening of the gumming and the folding-back and pressing-on of the flap is automatically controlled in a letter sealing device.

9. The use according to one of claims 1 to 6, wherein the sealing liquid is used in a letter sealing device with a tank and a moistener wing, wherein the moistener wing is adapted for receiving sealing liquid from the tank and transferring received sealing liquid on the gumming of a postal item inserted into the letter sealing device.

10. The use according to one of claims 1 to 6, wherein the sealing liquid is used in a franking device with a letter sealing device with a tank and a moistener wing, wherein the moistener wing is adapted for receiving sealing liquid from the tank and transferring received sealing liquid on the gumming of a postal item inserted into the letter sealing device.

## Revendications

1. Utilisation d'un liquide à cacheter comprenant de l'eau, un agent d'humectage et un agent de pénétration, dans laquelle l'agent de pénétration peut être un ester d'alkyle C1-8 d'acides mono-, di- ou tricarboxyliques C1-8, dans laquelle l'acide carboxylique peut être optionnellement substitué en OH, en particulier substitué en OH dans la position α, ou est un éther C1-8 de polyols C1-8, dans laquelle au moins un groupe OH du polyol n'est pas éthérisé, et dans laquelle l'agent d'humectage est un agent d'humectage tensioactif pour cacheter des envois postaux, dans laquelle une languette pourvue d'une gomme d'une enveloppe d'envoi postal enveloppant l'envoi postal est humectée avec le liquide à cacheter, et dans laquelle la languette humectée est puis pliée vers l'arrière et poussée sur une surface opposée de l'enveloppe d'envoi postal ou est utilisée pour la fixation et/ou fermeture d'un emballage en carton, dans laquelle un ruban adhésif pourvu d'une gomme est humecté avec le liquide à cacheter, et dans laquelle le ruban adhésif humecté est appliqué sur l'emballage en carton.

2. Utilisation selon la revendication 1, dans laquelle l'agent de pénétration est choisi à partir du groupe consistant en lactate de méthyle, lactate d'éthyle, lactate de propyle, lactate d'isopropyle, diester d'acide malique, monoester d'acide malique, diester d'acide tartarique, monoester d'acide tartarique, éther d'éthylène glycol monométhyle, éther d'éthylène glycol monoéthyle, éther d'éthylène glycol monopropyle, éther d'éthylène glycol monobutyle, éther de diéthylène glycol monoéthyle, éther de diéthylène glycol monoéthyle, éther de diéthylène glycol monopropyle, éther de diéthylène glycol monobutyle.

3. Utilisation selon la revendication 1 ou 2, dans laquelle l'agent d'humectage est choisi à partir du groupe consistant en tensioactifs non ioniques, tensioactifs anioniques, tensioactifs cationiques, tensioactifs amphotériques, bétaïnes de ricin oléamide propyle.

4. Utilisation selon une des revendications 1 à 3, dans laquelle le liquide à cacheter en plus comprend un ou plusieurs des agents additionnels et/ou auxiliaires suivants:
a) stabilisateurs et/ou tampons,
b) colorants, et
c) substances aromatisantes.

5. Utilisation selon une des revendications 1 à 4 comprenant
a) 50 à 99,9 % en poids d'eau,
b) 0,1 à 50,0 % en poids d'agent de pénétration,
c) 0,01 à 10,0 % en poids d'agent d'humectage,
d) 0 à 10,0 % en poids de stabilisateurs et/ou tampons,
e) 0 à 5,0 % en poids de colorants,
f) 0 à 5,0 % en poids de substances aromatisantes, et
g) 0 à 10,0 % en poids d'agents auxiliaires habituels,
dans laquelle la somme des composants a) à f) est toujours 100 % en poids.

6. Utilisation selon une des revendications 1 à 5 comprenant
a) 70 à 99,0, en particulier 80 à 99,0 % en poids d'eau,
b) 1,0 à 30,0, en particulier 1,0 à 20, % en poids d'agent de pénétration,
c) 0,01 à 3,0, en particulier 0,01 à 1,0, % en poids d'agent d'humectage,
d) 0 à 3,0, en particulier 0,01 à 2,0, % en poids de stabilisateurs et/ou tampons,
e) 0 à 2,0, en particulier 0,01 à 1,0, % en poids de colorants,
f) 0 à 1,0, en particulier 0,01 à 0,1 % en poids de substances aromatisantes et
g) 0 à 1,0, en particulier 0 à 0,1 % en poids de biocides et/ou fongicides et/ou d'autres agents auxiliaires habituels,
dans laquelle la somme des composants a) à f) est toujours 100 % en poids.

7. Utilisation selon une des revendications 1 à 6, dans laquelle l'envoi postal est une lettre et l'enveloppe d'envoi postal est une enveloppe de lettre.

8. Utilisation selon une des revendications 1 à 7, dans laquelle l'humectage de la gomme et le pliage et pressage de la languette est commandé automatiquement dans une machine à cacheter les lettres.

9. Utilisation selon une des revendications 1 à 6, dans laquelle le liquide à cacheter est utilisé dans un dispositif à cacheter les lettre avec un réservoir et un bras d'humectage, dans laquelle le bras d'humectage est agencé à recevoir le liquide à cacheter à partir du réservoir et transposer le liquide à cacheter reçu sur la gomme d'un envoi postal placé dans le dispositif à cacheter les lettres.

10. Utilisation selon une des revendications 1 à 6, dans laquelle le liquide à cacheter est utilisé dans un dispositif d'affranchissement comprenant un dispositif à cacheter les lettres avec un réservoir et un bras d'humectage, dans laquelle le bras d'humectage est agencé pour recevoir le liquide à cacheter à partir du réservoir et à transposer le liquide à cacheter reçu sur la gomme d'un envoi postal placé dans le dispositif à cacheter les lettres.
